# EUROPEAN PATENT APPLICATION

(11) **EP 4 762 986 A1**
(43) Date of publication of application: **24.06.2026**
(21) Application number: 24221327.0
(22) Date of filing: 18.12.2024
(51) Int. Cl.: A45C 11/00, A61L 12/08, G02C 13/00

(54) **LEAK-PROOF CONTACT LENS CASE**

(71) Applicant: YONG DA LI TECHNOLOGY CO., LTD., Taichung City (TW)
(72) Inventor: WANG, Chiang-Chuan, 50648 CHANGHUA COUNTY (TW)
(74) Representative: Lang, Christian

(57) **Abstract**

A leak-proof contact lens case is provided. The contact lens case consists of a main body, a lid, and a contact lens holder. The main body is provided with an opening, a receptacle, and a first fitting part. The lid has an outer upper surface, an inner upper surface, a rim, and a second fitting part. A vent and a tube are arranged in the middle of the inner upper surface. A first threaded portion is formed on the inner periphery of the tube. The vent comprises a first hole segment penetrating the inner upper surface and a second hole segment penetrating the outer upper surface, whereby the first hole segment is pinhole-sized and the second hole segment is distinctly larger than the first hole segment. The contact lens holder is provided with a base and two storage covers, whereby a shank protrudes from the centre of one end of the base and is provided with a second threaded portion on its outer periphery.

## Description

### BACKGROUND OF INVENTION

### 1. Field of Invention

The present invention relates to a leak-proof contact lens case in which contact lenses are placed, rinsed, and disinfected.

### 2. Description of the Related Art

Contact lenses are lenses that are worn directly and attached to the cornea of the eye. They can be used to correct vision problems, protect the eyes, slow down the progression of myopia (orthokeratology lenses). With the launch of colored contact lenses (circle contact lenses) that also have makeup functions, contact lenses are more widely used.

However, since the contact lens is in direct contact with the cornea when worn, cleaning after wearing is particularly important. Conventionally, contact lenses are placed in a cleaning solution in a contact lens case (or contact lens cleaning box), rinsed, and disinfected. The contact lens case is also used to store and protect the contact lenses during transport. Since the contact lenses will produce reaction gas when soaked in the solution, it is known that common contact lens containers/cases are provided with a vent through which the reaction gas can be discharged.

The conventional structure as mentioned above still has the following problems in practical application. Not only gas but also the solution contained in the contact lens container/case may run out through the vent. When the solution continues to overflow to such an extend that the solution in the contact lens container/case is insufficient to fully soak the contact lens, the contact lens will gradually dry out and be damaged. This phenomenon occurs quite often when the contact lens container/case is placed and dumped or carried (such as placing the contact lens container/case in a bag). Therefore, there is a need for improving the structure of such contact lens containers/cases.

### SUMMARY OF THE INVENTION

An objective of present invention is to provide a leak-proof contact lens case to prevent leakage.

To achieve these and other objects of the present invention, a leak-proof contact lens case is provided. The contact lens case consists of a main body, a lid, and a contact lens holder. The main body has an opening and a receptacle formed inside the main body for receiving the contact lens holder and a cleaning solution. A first fitting part is arranged at the opening. The lid has an outer upper surface, an inner upper surface, and a rim. The rim is provided with a second fitting part. A vent and a tube are arranged in the middle of the inner upper surface in such a way that they communicate with each other. A first threaded portion is formed on the inner periphery of the tube. The vent comprises a first hole segment penetrating the inner upper surface and a second hole segment penetrating the outer upper surface, whereby the first hole segment is pinhole-sized and the second hole segment is distinctly larger than the first hole segment to such an extent that the vent is cone-shaped and the cone widens from the first hole segment to the second hole segment gradually. The contact lens holder is provided with a base and two storage covers, whereby the two storage covers are swivel-mounted to both sides of the base and arranged for storing the contact lenses. A shank protrudes from the centre of one end of the base and is provided with a second threaded portion on its outer periphery. The contact lens holder is smoothly mounted to the tube of the lid by screwing the first threaded portion and the second threaded portion together. When the contact lens holder is placed in the receptacle containing the solution, the lid is to be put on the main body to seal the opening. In the rinsing and bathing process, a reactive gas is produced. The reactive gas that has no surface tension will then flow from the gaps between the first threaded portion and the second threaded portion and be discharged through the vent. The solution that has surface tension of liquid can be surely blocked and retained within the main body and the lid by the first hole segment with small clearance. Thus, leaks of the cleaning solution can be prevented.

The leak-proof contact lens case according to the present invention has following advantages. Due to the advantageous structural arrangement of the screw gaps between the first threaded portion and the second threaded portion in combination of the difference of the clearances of the first hole segment and the second hole segment, only reactive gas without surface tension can be discharged. Even when the contact lens case is overturned or entirely turned upside down, the cleaning solution with surface tension can still be surely blocked and retained within the main body and the lid by the first hole segment with small clearance, ensuring an absolute leakage protection. Thus, a safe storage of contact lenses in the main body and the lid and a sufficient amount of the cleaning solution can be achieved with the leak-proof contact lens case according to the invention.

Other objects, advantages, and novel features of invention will become more apparent from the following detailed description when taken in conjunction with the accompanying drawings.

### BRIEF DESCRIPTION OF DRAWINGS

FIG. 1 is a perspective view of a first preferred embodiment of a leak-proof contact lens case according to the present invention in an assembled state.
FIG. 2 is an explosive view of the contact lens case shown in Fig. 1.
FIG. 3 shows schematically the security lock shown in Fig. 1 in an assembled state in a sectional view.
FIG. 4 shows schematically the gas discharge of the first preferred embodiment.
FIG. 5 shows schematically the contact lens case of the first preferred embodiment in an overturned state without solution leakage.
FIG. 6 shows a perspective view of a second preferred embodiment of a leak-proof contact lens case according to the present invention.
FIG. 7 shows schematically the gas discharge of the second preferred embodiment.
FIG. 8 shows schematically a third preferred embodiment of a leak-proof contact lens case according to the present invention.

### DETAILED DESCRIPTION OF THE PREFERRED EMBODIMENT

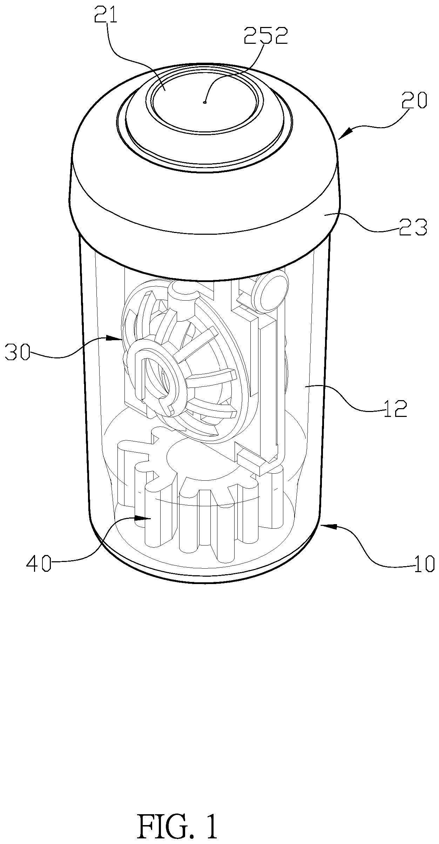

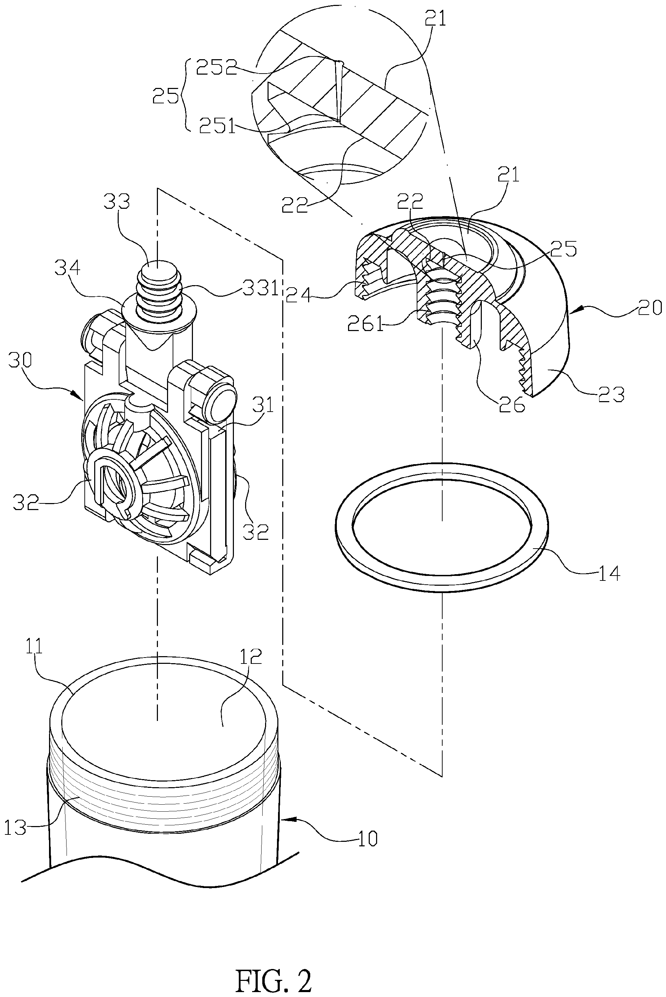

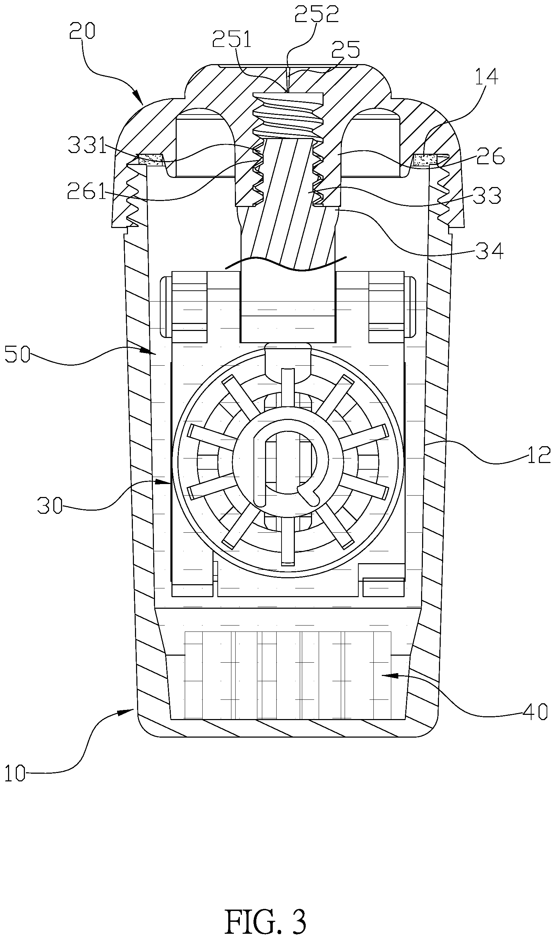

Please refer to FIG. 1 to 4, a leak-proof contact lens case is provided. The contact lens case consists of a main body 10, a lid 20, and a contact lens holder 30. The main body 10 has an opening 11 and a receptacle 12 formed inside the main body 10 for receiving the contact lens holder 30 and a cleaning solution 50. A first fitting part 13 is arranged at the opening 11. The lid 20 has an outer upper surface 21, an inner upper surface 22, and a rim 23. The rim 23 is provided with a second fitting part 24. A vent 25 and a tube 26 are arranged in the middle of the inner upper surface 22 in such a way that they communicate with each other. A first threaded portion 261 is formed on the inner periphery of the tube 26. The vent 25 comprises a first hole segment 251 penetrating the inner upper surface 22 and a second hole segment 252 penetrating the outer upper surface 21, whereby the first hole segment 251 is pinhole-sized and the second hole segment 252 is distinctly larger than the first hole segment 251 to such an extent that the vent 25 is cone-shaped and the cone widens from the first hole segment 251 to the second hole segment 252 gradually. The contact lens holder 30 has a base 31 on whose both sides a storage cover 32 for storing a contact lens is swivel-mounted respectively. A shank 33 protrudes from the centre of one end of the base 31 and is provided with a second threaded portion 331 on its outer periphery. The contact lens holder 30 is smoothly mounted to the tube 26 of the lid 20 by screwing the first threaded portion 261 and the second threaded portion 331 together. When the contact lens holder 30 is placed in the receptacle 12 containing the solution 50, the lid 20 is to be put on the main body 10 to seal the opening 11. In the rinsing and bathing process, a reactive gas 51 is produced. The reactive gas 51 that has no surface tension will then flow from the gaps between the first threaded portion 261 and the second threaded portion 331 and be discharged through the vent 25. The solution 50 that has surface tension of liquid can be surely blocked and retained within the main body 10 and the lid 20 by the first hole segment 251 with small clearance. Thus, leaks of the cleaning solution 50 can be prevented.

According to the invention, the main body 10 is a transparent cylinder-shaped container.

According to the invention, the first fitting part 13 is an external screw thread and the second fitting part 24 is an internal screw thread, so that the lid 20 can be connected with the main body 10 by screwing the second fitting part 24 with the first fitting part 13, sealing the opening 11 accordingly.

According to the invention, a sealing ring 24 is clamped between the main body 10 and the lid 20.

According to the invention, a stop collar 34 is formed extended at the shank 33. When the shank 33 and the tube 26 are screwed together, the stop collar 34 abuts on the tail-end of the tube 26, allowing the reactive gas 51 flowing from the contact gap between the stop collar 34 and the tube 26 into the tube 26 and then being discharged through the vent 25.

According to the invention, the stop collar 34 is a disc.

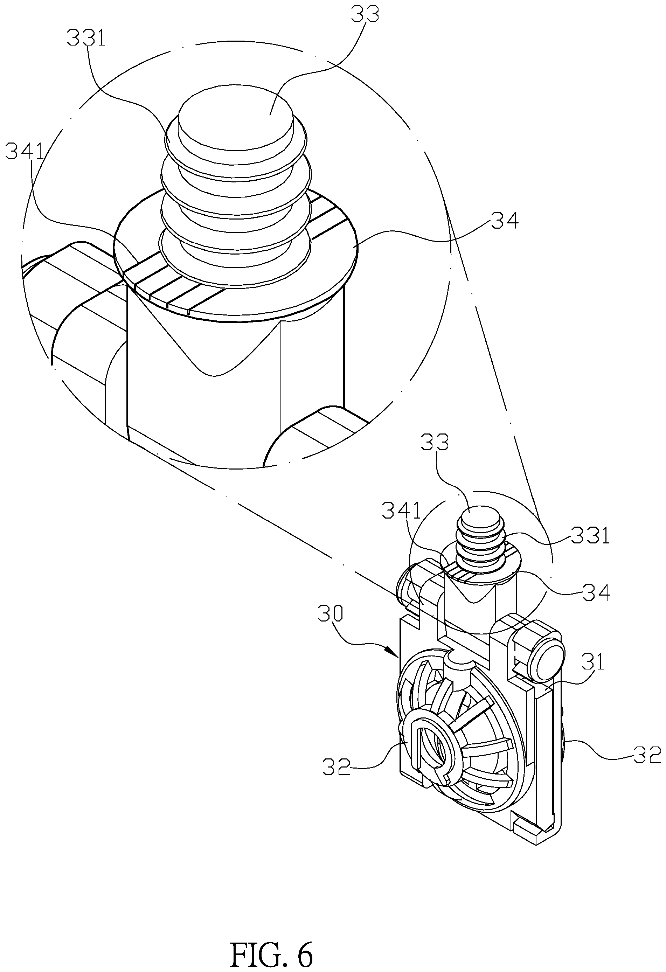

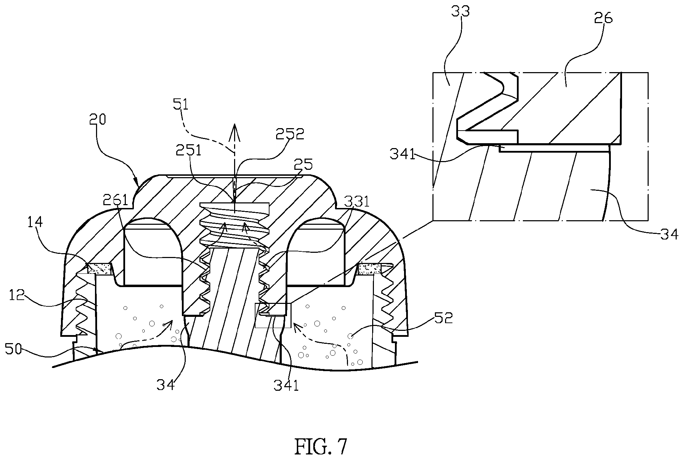

According to the invention, the stop collar 34 is a disc and provided with at least one exhaust groove 341 (see also Fig. 6), enabling the reactive gas 51 entering the tube 26 through the exhaust groove 341 at an accelerated tempo (see Fig. 7) and then being discharged.

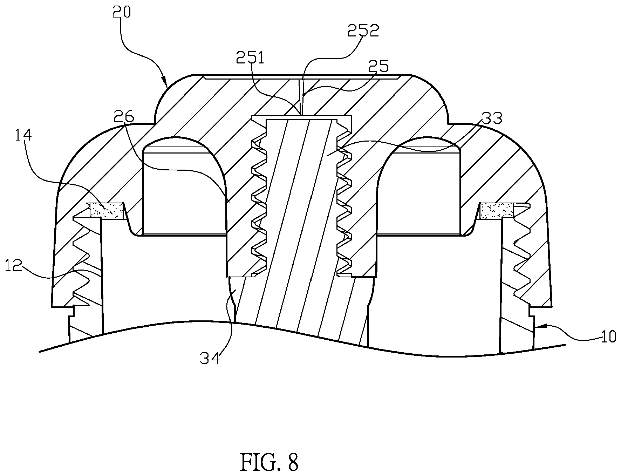

According to the invention, the shank 33 is a short stud (see Fig. 1 bis 7), can, however, also be a long stud (see Fig. 8), so that the contact lens holder 30 can be mounted to the tube 26 smoothly regardless of the length of the shank 33, achieving a reliable gas discharge and a reliable leakage protection.

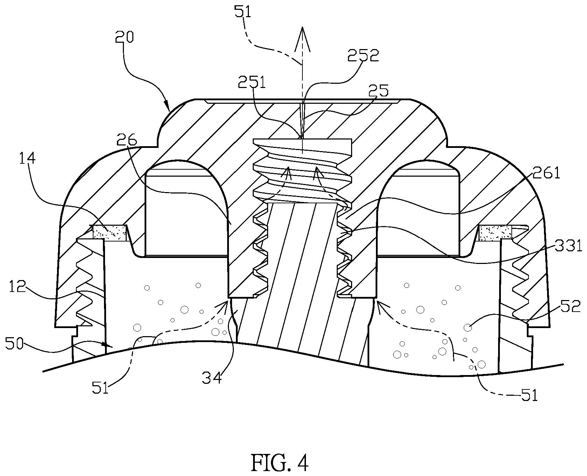

According to the invention, a reactor 40 is placed in the receptacle 12 containing the solution 50. When the reactor 40 gets in contact with the solution 50, a lot of cleaning air bubbles 52 will be produced and a reactive gas 51 will thereby be produced (see Fig. 4)

According to the invention, the reactor 40 is a platinum reactor.

The usage of the contact lens case is described as follows. Contact lenses are to be put in the storage covers 32 of the contact lens holder 30 in such a way that the contact lenses can be placed in the cleaning solution 50 in the main body 10 together with the contact lens holder 30. In the bathing progress, a neutralization in which the cleaning solution 50 reacts with the reactor 40 results in producing a reactive gas 51 and a lot of cleaning air bubbles 52. Thus, the contact lenses are rinsed with the cleaning air bubbles 52 and disinfected at the same time in the bathing process. Since a liquid has surface tension and gas has no surface tension, the produced reactive gas 51 will flow from the first hole segment 251 with small clearance and then be discharged through the vent 25 (see also Fig. 4). On the contrary, the cleaning solution 50 with surface tension will be surely blocked and retained within the main body 10 and the lid 20 by the first hole segment 251 with small clearance, thereby successfully preventing the solution 50 from leaking.

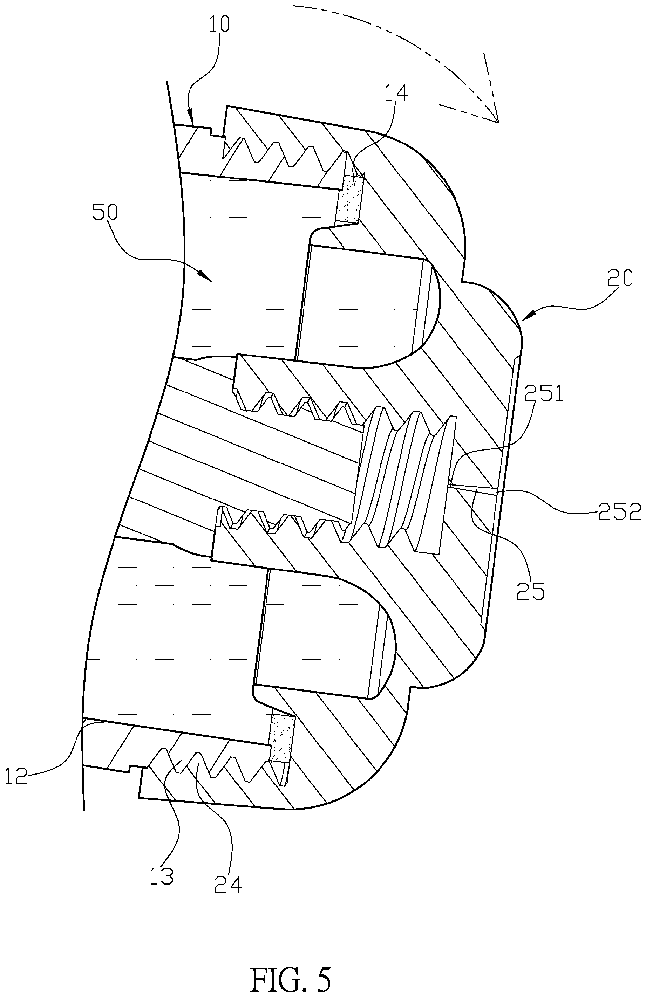

The leak-proof contact lens case according to the present invention has following advantages. Due to the advantageous structural arrangement of the screw gaps between the first threaded portion 261 and the second threaded portion 331 in combination of the difference of the clearances of the first hole segment 251 and the second hole segment 252, only reactive gas 51 without surface tension can be discharged (see Fig. 4). Even when the contact lens case is overturned or entirely turned upside down, the cleaning solution 50 with surface tension can still be surely blocked and retained within the main body 10 and the lid 20 by the first hole segment 251 with small clearance, ensuring an absolute leakage protection (see Fig. 5). Thus, a safe storage of contact lenses in the main body 10 and the lid 20 and a sufficient amount of the cleaning solution 50 can be achieved with the leak-proof contact lens case according to the invention.

Although the present invention has been explained in relation to its preferred embodiment, it is to be understood that many other possible modifications and variations can be made without departing from the spirit and scope of invention as hereinafter claimed.

## Claims

1. A leak-proof contact lens case, comprising a main body, a lid, and a contact lens holder, **characterized in**
**that** the main body has an opening and a receptacle formed inside the main body for receiving the contact lens holder and a cleaning solution, wherein a first fitting part is arranged at the opening;
**that** the lid has an outer upper surface, an inner upper surface, and a rim, wherein the rim is provided with a second fitting part, a vent and a tube are arranged in the middle of the inner upper surface in such a way that they communicate with each other, wherein a first threaded portion is formed on the inner periphery of the tube, wherein the vent comprises a first hole segment penetrating the inner upper surface and a second hole segment penetrating the outer upper surface, whereby the first hole segment is pinhole-sized and the second hole segment is distinctly larger than the first hole segment to such an extent that the vent is cone-shaped and the cone widens from the first hole segment to the second hole segment gradually; and
**that** the contact lens holder has a base on whose both sides a storage cover for storing a contact lens is swivel-mounted respectively, wherein a shank protrudes from the centre of one end of the base and is provided with a second threaded portion on its outer periphery, wherein the contact lens holder is smoothly mounted to the tube of the lid by screwing the first threaded portion and the second threaded portion together, wherein the lid is to be put on the main body to seal the opening when the contact lens holder is placed in the receptacle containing the solution, enabling a reactive gas that is produced in the rinsing and bathing process and has no surface tension to flow from the gaps between the first threaded portion and the second threaded portion and be discharged through the vent, whereas the solution that has surface tension of liquid can be surely blocked and retained within the main body and the lid by the first hole segment with small clearance.

2. The leak-proof contact lens case as claimed in claim 1, wherein the main body is a transparent cylinder-shaped container.

3. The leak-proof contact lens case as claimed in claim 1, wherein the first fitting part is an external screw thread and the second fitting part is an internal screw thread, so that the lid can be connected with the main body by screwing the second fitting part with the first fitting part, sealing the opening accordingly.

4. The leak-proof contact lens case as claimed in claim 1, wherein a sealing ring is clamped between the main body and the lid.

5. The leak-proof contact lens case as claimed in claim 1, wherein a stop collar is formed extended at the shank and abuts on the tail-end of the tube when the shank and the tube are screwed together, allowing the reactive gas flowing from the contact gap between the stop collar and the tube into the tube and then being discharged through the vent.

6. The leak-proof contact lens case as claimed in claim 5, wherein the stop collar is a disc.

7. The leak-proof contact lens case as claimed in claim 5, wherein the stop collar is a disc and provided with at least one exhaust groove.

8. The leak-proof contact lens case as claimed in claim 1, wherein a reactor is placed in the receptacle containing the solution.

9. The leak-proof contact lens case as claimed in claim 8, wherein the reactor is a platinum reactor.
